# EUROPÄISCHE PATENTSCHRIFT

(11) **EP 0 816 313 B1**
(45) Veröffentlichungstag und Bekanntmachung des Hinweises auf die Patenterteilung: **18.04.2001**
(21) Anmeldenummer: 97109998.1
(22) Anmeldetag: 19.06.1997
(51) Int. Cl.: C07C 17/16, C07C 17/38, C07C 17/383, C07C 17/386, C07C 19/03, C07C 41/09, C07C 41/42, C07C 43/04

(54) **Verfahren zur Herstellung und Auftrennung eines Gemischs aus Dimethylether und Chlormethan mit Methanol als Extraktionsmittel**
Process for the preparation and separation of a mixture of dimethyl ether and chloromethane using methanol as the extractant
Procédé pour la préparation et la séparation d'un mélange d'éther diméthylique et de chlorométhane au moyen de méthanol comme agent d'extraction

(30) Priorität: 25.06.1996 DE 19625282
(43) Veröffentlichungstag der Anmeldung: 07.01.1998
(73) Patentinhaber: HOECHST AKTIENGESELLSCHAFT, 65929 Frankfurt am Main (DE)
(72) Erfinder: Roth, Peter, Dl., 65817 Eppstein (DE); Leistner, Erhard, Dl., 35619 Braunfels (DE); Wendel, Wolfgang, Dr., 65779 Kelkheim (DE)
(74) Vertreter: Schweitzer, Klaus, Dr.

(56) Entgegenhaltungen:
- EP-A- 0 039 001
- EP-A- 0 124 078
- US-A- 2 421 441
- US-A- 4 220 609
- US-A- 5 092 966
- DATABASE WPI Section Ch, Week 8036 Derwent Publications Ltd., London, GB; Class E16, AN 80-62426C XP002040756 & DD 142 183 A (BERGER K H) , 11.Juni 1980

## Beschreibung

Die vorliegende Erfindung betrifft ein Verfahren zur Herstellung eines Gemischs aus Dimethylether und Chlormethan, sowie dessen Auftrennung mittels Extraktivdestillation mit Methanol als Extraktionsmittel.

Chlormethan besitzt technische Bedeutung als Ausgangsprodukt für die Herstellung von Fluorchlorkohlenwasserstoffen, die dann als Treibgase in Aerosolpackungen Verwendung finden. Dimethylether wird im zunehmendem Maße als Treibmittel in Aerosolpackungen eingesetzt, da es halogenfrei ist, und somit ein geringes Ozonabbaupotential aufweist.

Beide Verbindungen entstehen gemäß den Formelzeilen (I) und (II) bei der Umsetzung von Methanol mit HCI, die zur Reaktionsbeschleunigung technisch häufig an Y-Al₂O₃-Kontakten durchgeführt wird:

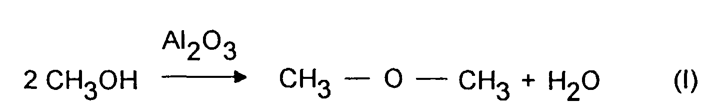

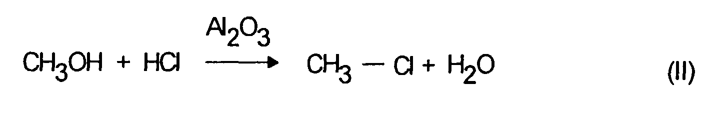

Bislang war es nach dem Stand der Technik üblich, den bei der Chlormethanherstellung anfallenden Dimethylether als Abfall anzusehen, und ihn durch Hydrolyse mit Schwefelsäure zu beseitigen. Da Dimethylether wegen seiner Verwendbarkeit als Treibmittel aber ein wertvolles Produkt ist, bestand der Bedarf für ein Verfahren, das den Zwangsanfall an Dimethylether technisch nutzbar macht.

Die Mischung aus Dimethylether und Chlormethan ist nach dem Stand der Technik destillativ nicht aufarbeitbar, da die Siedepunkte der Komponenten sehr nahe beieinander liegen (Dimethyether Kp= - 24,9 °C, Chlormethan Kp= - 23,7 °C), und die Komponenten außerdem ein Azetrop bilden.

DD-142 183 beschreibt ein Verfahren zur Trennung von Wasser, Methanol und den Chlormethanen Methylenchlorid, Chloroform und Tetrachlormethan durch Extraktivdestillation.

Die Aufgabe bestand somit darin, ein Herstellungs- und Trennverfahren zu entwickeln, das die Produktion sowohl reinen Dimethylethers als auch reinen Chlormethans ermöglicht.

Diese Aufgabe wurde erfindungsgemäß durch ein zweistufiges Syntheseverfahren mit angeschlossenem dreistufigen Aufarbeitungsverfahren gelöst, wobei die Aufarbeitung eine Extraktivdestillation mit Methanol umfaßt.

Gegenstand der Erfindung ist somit ein Verfahren zur Herstellung von Dimethylether und Chlormethan durch Umsetzung von Methanol mit HCl und Auftrennung des entstehenden Gemisches, dadurch gekennzeichnet, daß man
a) Methanol mit einem Überschuß an HCI umsetzt,
b) das in Schritt a) erhaltene, Wasser enthaltende Gemisch mit einem Überschuß an Methanol umsetzt,
c) das in Schritt b) erhaltene Gemisch einer ersten Destillationskolonne zuführt,
d) vom Sumpf der ersten Destillationskolonne Wasser abzieht,
e) das am Kopf der ersten Destillationskolonne austretende, Methanol, Dimethylether und Chlormethan enthaltende Gemisch abzieht,
f) das in Schritt e) erhaltene Gemisch einer Extraktionsdestillationskolonne zuführt,
g) in den oberen Teil der Extraktivdestillationskolonne Methanol als Extraktionsmittel aufgibt,
h) vom Sumpf der Extraktivdestillationskolonne ein Gemisch aus Methanol und Dimethylether abzieht,
i) vom Kopf der Extraktivdestillationskolonne Chormethan ableitet,
k) das in Schritt h) entnommene Gemisch einer zweiten Destillationskolonne zuführt,
l) vom Sumpf der zweiten Destillationskolonne Methanol abzieht,
m) am Kopf der zweiten Destillationskolonne Dimethylether entnimmt,
n) einen Teil des in Schritt l) erhaltenen Methanols den in den Schritten a) und b) beschriebenen Reaktionen zuführt, und
o) den verbleibenden Teil des Schritt l) erhaltenen Methanols der in Schritt g) beschrieenen Methanolaufgabe in den oberen Teil der Extraktivdestillationskolonne zuführt.

Die Verwendung eines Katalysators für die in den Schritten a) und b) beschriebene Veresterungsreaktion ist bevorzugt. Es kommen Υ-Al₂O₃-Kontakte in Frage.

Bevorzugt geschieht die in Schritt c) beschriebene Gemischförderung dadurch, daß man gasförmig anfallendes Gemisch in einem Kondensator verflüssigt, das Kondensat mit einer Pumpe fördert, und es vor dem Eintritt in die erste Destillationskolonne wieder verdampft.

Vorzugsweise wird die Exktrativdestillation bei Drucken zwischen 1 und 25 bar durchgeführt. Alle verwendeten Kolonnen können von einer beliebigen geeigneten Bauart sein, bevorzugt ist die Verwendung von Füllkörper- oder Packungskolonnen.

In einer Ausführungsform der Erfindung wird ein Teil des in Schritt m) erhaltenen Produktstromes entnommen und der in den Schritten a) und b) beschriebenen Reaktion zugeführt. Diese Ausführungsform erlaubt mittels einer Gleichgewichtsverschiebung eine Beeinflussung des Verhältnisses der produzierten Mengen an Chlormethan und Dimethylether.

Figur I zeigt ein Fließschema für eine bevorzugte Ausführungsform des erfindungsgemäßen Verfahrens, das im folgenden näher erläutert wird. Einem zweistufigen Veresterungsreaktor (6) wird dampfförmiges Methanol (1) über Leitung (3) zugeführt. Außerdem wird gasförmiges HCl (2) über Leitung (5) in den Reaktor (6) eingespeist. Der zweistufige Veresterungsreaktor kann in Form eines zweistufigen Rohrreaktors ausgeführt sein. In der ersten Stufe werden HCl und Methanol in den Reaktor aufgegeben. Die Mengen der Reaktanden sind so bemessen, daß die Stoffmenge an HCl bis zu 20% über der stöchiometrisch zur Reaktion mit dem Methanol nötigen Stoffmenge liegt. Das Gemisch aus HCl und Methanol durchströmt die erste Stufe des Reaktors, die mit einem Katalysator beaufschlagt sein kann. Im Fall einer Gasphasenreaktion verläuft die Reaktion exotherm, so daß eine Kühlung für beide Stufen des Reaktors vorzusehen ist. Das die erste Stufe des Reaktors verlassende Gemisch aus Chlormethan, Wasser und HCl wird mit einer Menge an Methanol gemischt, die bis zu 20% über der Stoffmenge liegt, die stöchiometrisch zur Reaktion mit dem im Gemisch enthaltenen HCl nötig ist. Das Gemisch durchströmt die zweite Stufe des Reaktors, die ebenfalls mit einem Katalysator beaufschlagt sein kann. Die Reaktion kann mit flüssigen oder gasförmigen Reaktanden durchgeführt werden. Im Reaktor (6) laufen die in den Formelzeilen (I) und (II) angegebenen Reaktionen ab. Das anfallende Reaktionsgemisch, das aus Dimethylether, Chlormethan, Wasser und Methanol besteht, verläßt den Reaktor (6) über Leitung (10), und kann im Kondensator (11) kondensiert werden. Von dort gelangt es über die Leitung (12) zur Pumpe (13), dann über Leitung (14) zum Verdampfer (15). Das verdampfte Reaktionsgemisch wird über Leitung (16) in die erste Destillationskolonne (17) eingespeist. In der ersten Destillationskolonne (17) erfolgt die Abtrennung des Reaktionswassers (25) von den übrigen Komponenten, also Dimethylether, Chlormethan und Methanol. Das Reaktionswasser (25) fällt im Sumpf der ersten Destillationskolonne (17) an, und wird über Leitung (24) abgeführt. Der in der ersten Destillationskolonne (17) anfallende, aus Dimethylether, Chlormethan und Methanol bestehende Kopfdampf gelangt über Leitung (18) gegebenenfalls in den Kondensator (19). Ein Teil des Kopfproduktes der ersten Destillationskolonne (17) kann über Leitung (21) auf den Kopf der ersten Destillationskolonne (17) zurück geführt werden, der verbleibende Teil gelangt über Leitung (20) zur Extraktivdestillationskolonne (26). Alternativ kann der Produktstrom auch als Dampf in die Extraktivdestillationskolonne (26) eingespeist werden. In der Extraktivdestillationskolonne (26) erfolgt die Trennung zwischen Dimethylether und Chlormethan. Im oberen Teil der Extraktivdestillationskolonne (26) wird über Leitung (56) Methanol als Extraktionsmittel aufgegeben. Bevorzugt ist es, Methanol mit einer Temperatur von 5 bis 50 °C aufzugeben. Dimethylether löst sich in Methanol und wird im Sumpf niedergeschlagen. Das aus Dimethylether und Methanol bestehende Sumpfprodukt der Extraktivdestillationskolonne (26) wird über Leitung (34), Pumpe (35), Leitung (36), gegebenenfalls den Wärmetauscher (37) und die Leitung (38) in die zweite Destillationskolonne (39) eingespeist. Als Kopfprodukt der Extraktivdestillationskolonne (26) entnimmt man über Leitung (27) reines Chlormethan, das im Kondensator (28) verflüssigt werden kann. Dieses Kopfprodukt wird über Leitung (29) abgeführt, und kann teilweise über die Leitung (30) der Extraktivdestillationskolonne (26) wieder zugeführt werden, sonst wird es ganz oder teilweise über Leitung (31) dem Produktlager (32) zugeführt. In der zweiten Destillationskolonne (39) erfolgt die Trennung zwischen Dimethylether und Methanol. Als Kopfprodukt fällt in der zweiten Destillationskolonne (39) reiner Dimethylether an. Dieser kann über Leitung (40) dem Kondensator (41) zugeführt werden. Dieses Kopfprodukt kann über die Leitungen (42) und (43) teilweise zur zweiten Destillationskolonne (39) zurückgeführt werden, sonst wird es ganz oder teilweise über die Leitungen (42), (44) und (46) dem Produktlager (47) zugeführt.

Alternativ kann ein Dimethylether-Teilstrom von Leitung (44) über Leitung (45) dem Veresterungsreaktor (6) zugeführt werden. Die Beschickung des Veresterungsreaktors (6) mit Dimethylether ermöglicht über eine Gleichgewichtsbeeinflussung die Änderung des Produktverhältnisses von Dimethylether zu Chlormethan.

Als Sumpfprodukt der zweiten Destillationskolonne (39) fällt reines Methanol an. Es wird über Leitung (49) einer Pumpe (50), und von dort über Leitung (52) teilweise über Leitung (51) dem Verdampfer (57) zugeführt, und über Leitung (58) in den Veresterungsreaktor (6) eingespeist. Der andere Methanol-Teilstrom wird über Leitung (53), gegebenenfalls den Wärmetauscher (37), Leitung (54), den Kühler (55) und Leitung (56) dem oberen Teil der Extraktivdestillationskolonne (26) zugeführt. Über Leitung (59) wird zum Ausgleich von Verlusten frisches Methanol (60) in die Anlage eingespeist. Die Sümpfe der drei Destillationskolonnen (17), (26) und (39) werden jeweils mit den Verdampfern (23), (33) und (48) beheizt.

Die folgende Tabelle gibt eine Übersicht über die in den Kolonnen auftretenden Kopf- und Sumpftemperaturen im bevorzugten Druckbereich (Temperaturen in °C).

| | 1 bar | | 25 bar | |
|---|---|---|---|---|
| Kolonne | Kopf | Sumpf | Kopf | Sumpf |
| (17) | - 20 | 100 | 70 | 224 |
| (26) | - 24 | 80 | 90 | 180 |
| (39) | - 25 | 64 | 85 | 180 |

### Beispiel:

Einem Veresterungsreaktor (6) werden 7,1 t/h Methanol und 5,3 t/h HCI zugeführt. Die zweistufige Reaktion bei 250 °C und einem Druck von 4 bar unter Verwendung eines Υ-Al₂O₃-Katalysators bewirkt die Entstehung eines Reaktionsgases folgender Zusammensetzung:

| | |
|---|---|
| Dimethylether | 1,8 t/h |
| Methanol | 0,01 t/h |
| Chlormethan | 7,3 t/h |
| H₂O | 3,29 t/h |

Dieses Reaktionsgas wird bei etwa 4 bar und einer tiefsten Temperatur von etwa 10 °C vollständig in einem Kondensator (11) verflüssigt. Das anfallende Kondensat wird mit der Pumpe (13) in den Verdampfer (15) gefördert.

Hier erfolgt die teilweise Verdampfung des Kondensats bei 10 bar und etwa 100 °C. Als Heizmedium kann hierfür die im Kondensator (11) freiwerdende Kondensationswärme verwendet werden. Das Dampf/Flüssigkeitsgemisch aus dem Verdampfer (15) wird vollständig in die erste Destillationskolonne (17) eingespeist. Diese wird ebenfalls bei einem Druck von 10 bar, sowie mit einer Kopftemperatur von 45°C und einer Sumpftemperatur von 177°C betrieben. Als Sumpfprodukt fallen etwa 3,3 t/h Reaktionswasser (25) an. Das Reaktionswasser ist frei von den übrigen Produkten und kann einer biologischen Wasserreinigung zugeführt werden. Das dampfförmige Kopfprodukt nach dem Kondensator (19) besteht somit aus:

| | |
|---|---|
| Dimethylether | 1,8 t/h |
| Methanol | 0,01 t/h |
| Chlormethan | 7,3 t/h |

mit geringem Anteil an Wasser (etwa 5 ppm H₂O).

Es wird über die Leitung (20) in die Extraktivdestillationskolonne (26) eingespeist. Der Dimethylether wird hier in 42 t/h Methanol als Extraktionsmittel gelöst. Da sich gleichzeitig im Extraktionsmittel eine gewisse Menge Chlormethan löst, wird dieses im unteren Teil der Extraktivdestillationskolonne (26) ausgestrippt. Die Sumpfbeheizung kann mittels Heizdampf über den Verdampfer (33) erfolgen. Als Sumpfprodukt der Extraktivdestillation fällt ein Gemisch, bestehend aus

| | |
|---|---|
| 1,8 t/h | Dimethylether |
| 42 t/h | Extraktions-Methanol |
| 0,01 t/h | Methanol-Überschuß aus der Reaktion |

an. Der Sumpf enthält etwa 1 ppm Chlormethan. Als Kopfprodukt der Extraktivdestillationskolonne (26) fallen 7,3 t/h Chlormethan mit einem Dimethylether-Anteil von weniger als 20 ppm an. Die Extraktivdestillationskolonne (26) wird ebenfalls bei einem Druck von 10 bar betrieben. Das Extraktionsmethanol muß möglichst kalt, vorzugsweise mit etwa 35 °C, über Leitung (56) eingespeist werden. Die Kopftemperatur der Extraktivdestillationskolonne (26) beträgt etwa 40 °C, was die Kondensation des Kopfproduktes mittels Rückkühlwasser ermöglicht. Die Sumpftemperatur beträgt etwa 124 °C, so daß die Beheizung mit Niederdruck-Dampf möglich ist.

Mit Hilfe der Pumpe (35) wird der Sumpfablauf der Extraktivdestillationskolonne (26) über den Wärmetauscher (37) vorgewärmt und in die zweite Destillationskolonne (39) eingespeist. Auch diese Kolonne wird bei 10 bar betrieben. Als Kopfprodukt fallen etwa 1,8 t/h flüssiger Dimethylether bei etwa 40 °C an. Der Anteil Chlormethan im Dimethylether liegt bei weniger als 20 ppm.

Das Sumpfprodukt aus der zweiten Destillationskolonne (39) hat eine Temperatur von 132°C und ist reines Methanol mit weniger als 20 ppm Dimethylether. Es besteht aus dem "Extraktions-Methanol" und dem "Überschuß-Methanol" aus der Reaktion. Das "Überschuß-Methanol" (0,01 t/h) wird über Leitung (51) zur Reaktion rückgeführt, während das "Extraktions-Methanol" über die Wärmetauscher (37) und (55) gekühlt wird, um erneut über Leitung (56) in die Extraktivdestillationskolonne (26) eingespeist zu werden.

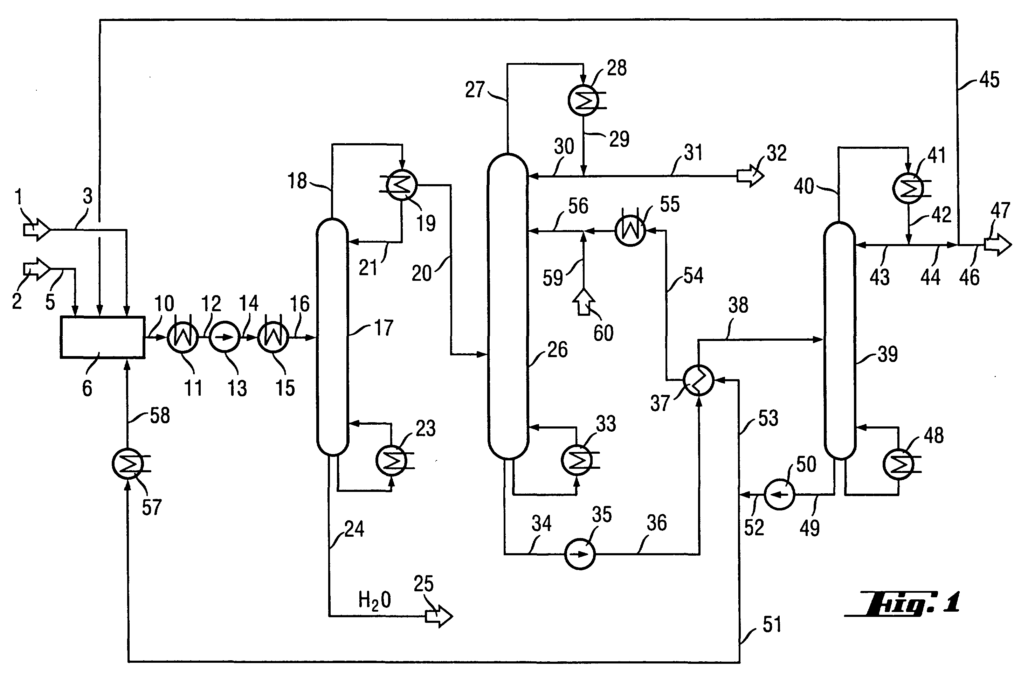

## Patentansprüche

1. Verfahren zur Herstellung von Dimethylether und Chlormethan durch Umsetzung von Methanol mit HCI und Auftrennung des entstehenden Gemisches, dadurch gekennzeichnet, daß man
a) Methanol mit einem Überschuß an HCI umsetzt,
b) das in Schritt a) erhaltene, Wasser enthaltende Gemisch mit einem Überschuß an Methanol umsetzt,
c) das in Schritt b) erhaltene Gemisch einer ersten Destillationskolonne zuführt,
d) vom Sumpf der ersten Destillationskolonne Wasser abzieht,
e) das am Kopf der ersten Destillationskolonne austretende, Methanol, Dimethylether und Chlormethan enthaltende Gemisch abzieht,
f) das in Schritt e) erhaltene Gemisch einer Extraktionsdestillationskolonne zuführt,
g) in den oberen Teil der Extraktivdestillationskolonne Methanol als Extraktionsmittel aufgibt,
h) vom Sumpf der Extraktivdestillationskolonne ein Gemisch aus Methanol und Dimethylether abzieht,
i) vom Kopf der Extraktivdestillationskolonne Chormethan ableitet,
k) das in Schritt h) entnommene Gemisch einer zweiten Destillationskolonne zuführt,
l) vom Sumpf der zweiten Destillationskolonne Methanol abzieht,
m) am Kopf der zweiten Destillationskolonne Dimethylether entnimmt,
n) einen Teil des in Schritt I) erhaltenen Methanols den in den Schritten a) und b) beschriebenen Reaktionen zuführt, und
o) den verbleibenden Teil des Schritt I) erhaltenen Methanols der in Schritt g) beschrieenen Methanolaufgabe in den oberen Teil der Extraktivdestillationskolonne zuführt.

2. Verfahren nach Anspruch 1, dadurch gekennzeichnet, daß ein Teil des in Schritt m) erhaltenen Produktstroms in den Schritten a)und b) beschriebenen Reaktionen zugeführt wird.

3. Verfahren nach einem der Ansprüch 1 oder 2, dadurch gekennzeichnet, daß die in den Schritten i) und m) erhaltenen Kopfprodukte vor deren weiterer Verwendung kondensiert werden.

4. Verfahren nach einem der Ansprüche 1 bis 3, dadurch gekennzeichnet, daß das in Schritt e) erhaltene Gemisch kondensiert wird, und inflüssiger Phase der Weiterverarbeitung zugeführt wird.

5. Verfahren nach einem der Ansprüche 1 bis 4, dadurch gekennzeichnet, daß während der in Schritt c) beschriebenen Zuführung gasförmig anfallendes Reaktionsgemisch zuerst kondensiert und dann wieder verdampft wird.

6. Verfahren nach einem der Ansprüche 1 bis 5, dadurch gekennzeichnet, daß die nach Schritt f) erfolgende Extraktivdestillation bei Drucken zwischen 1 und 25 bar durchgeführt wird.

7. Verfahren nach einem der Ansprüche 1 bis 6, dadurch gekennzeichnet, daß alle verwendeten Kolonnen als Füllkörper- oder Packungskolonnen ausgeführt werden.

8. Verfahren nach einem der Ansprüche 1 bis 7, dadurch gekennzeichnet, daß in Schritt g) das Extraktionsmethanol mit einer Temperatur zwischen 5 und 50 °C in die Extraktivdestillation eingespeist wird.

9. Verfahren nach einem der Ansprüche 1 bis 8, dadurch gekennzeichnet, daß man das in Schritt k) beschriebene Gemisch vor der Aufgabe in die zweite Destillationskolonne erwärmt, und daß man das in Schritt o) erwähnte Methanol vor der Aufgabe in die Extraktivdestillationskolonne abkühlt.

10. Verfahren nach Anspruch 9, dadurch gekennzeichnet, daß die Erwärmung des in Schritt k) beschriebenen Gemisches in einem Wärmetauscher erfolgt, und daß die Abkühung des in Schritt o) erwähnten Methanols ebenfalls in dem Wärmetauscher erfolgt, und daß man das Methanol in einem Kühler abkühlt.

11. Verfahren nach einem der Ansprüche 1 bis 10, dadurch gekennzeichnet, daß die in den Schritten a) und b) beschriebene Reaktion unter Zuhilfenahme eines Katalysators durchgeführt wird.

12. Verfahren nach einem der Ansprüche 1 bis 11, dadurch gekennzeichnet, daß die in den Schritten a) und b) genannten Eduktüberschüsse bis zu 20 % der zur Reaktion erforderlichen stöchiometrischen Mengen betragen.

13. Verfahren nach einem der Ansprüche 1 bis 11, dadurch gekennzeichnet, daß man
1) einen Teil des in Schritt e) erhaltenen Gemischs auf den Kopf der ersten Destillationskolonne zurückführt,
und/oder
2) das in Schritt l) erhaltene Produkt zum Teil auf den Kopf der Extraktivdestillationskolonne zurückführt,
und/oder
3) einen Teil des in Schritt m) erhaltenen Produkts auf den Kopf der zweiten Destillationskolonne zurückführt.

## Claims

1. A process for the preparation of dimethyl ether and chloromethane by reacting methanol with HCl and fractionating the resulting mixture, which comprises
a) reacting methanol with an excess of HCl,
b) reacting the water-containing mixture obtained in step a) with an excess of methanol,
c) feeding the mixture obtained in step b) to a first distillation column,
d) taking off water from the bottom product from the first distillation column,
e) taking off the mixture containing methanol, dimethyl ether and chloromethane which emerges at the top of the first distillation column,
f) feeding the mixture obtained in step e) to an extractive distillation column,
g) adding methanol as extractant in the upper part of the extractive distillation column,
h) taking off a mixture of methanol and dimethyl ether from the bottom product of the extractive distillation column,
i) drawing off chloromethane from the top of the extractive distillation column,
k) feeding the mixture taken off in step h) to a second distillation column,
l) taking off methanol from the bottom product of the second distillation column,
m) taking off dimethyl ether at the top of the second distillation column,
n) feeding part of the methanol obtained in step 1) into the reactions described in steps a) and b), and
o) feeding the remaining part of the methanol obtained in step l) to the addition, described in step g), of methanol into the upper part of the extractive distillation column.

2. The process as claimed in claim 1, wherein part of the product stream obtained in step m) is fed into the reactions described in steps a) and b).

3. The process as claimed in either of claims 1 and 2, wherein the top products obtained in steps i) and m) are condensed before further use thereof.

4. The process as claimed in any of claims 1 to 3, wherein the mixture obtained in step e) is condensed and fed in liquid phase to further processing.

5. The process as claimed in any of claims 1 to 4, wherein the reaction mixture produced as gas during the feeding described in step c) is first condensed and then vaporized again.

6. The process as claimed in any of claims 1 to 5, wherein the extractive distillation taking place after step f) is carried out under pressures between 1 and 25 bar.

7. The process as claimed in any of claims 1 to 6, wherein all the columns used are designed as packed columns.

8. The process as claimed in any of claims 1 to 7, wherein in step g), the extraction methanol is fed at a temperature between 5 and 50°C into the extractive distillation.

9. The process as claimed in any of claims 1 to 8, wherein the mixture described in step k) is heated before addition to the second distillation column, and wherein the methanol mentioned in step o) is cooled before addition to the extractive distillation column.

10. The process as claimed in claim 9, wherein the heating of the mixture described in step k) takes place in a heat exchanger, and wherein the cooling of the methanol mentioned in step o) likewise takes place in the heat exchanger, and wherein the methanol is cooled in a cooler.

11. The process as claimed in any of claims 1 to 10, wherein the reaction described in steps a) and b) is carried out with the aid of a catalyst.

12. The process as claimed in any of claims 1 to 11, wherein the excesses of precursors mentioned in steps
a) and b) are up to 20 % of the stoichiometric amounts required for the reaction.

13. The process as claimed in any of claims 1 to 11, wherein
1) part of the mixture obtained in step e) is returned to the top of the first distillation column,
and/or
2) the product obtained in step 1) is partly returned to the top of the extractive distillation column,
and/or
3) part of the product obtained in step m) is returned to the top of the second distillation column.

## Revendications

1. Procédé pour la préparation d'éther diméthylique et de chlorométhane par mise en réaction du méthanol avec HCl et fractionnement du mélange résultant, caractérisé en ce que
a) on fait réagir du méthanol avec un excès de HCl,
b) on fait réagir avec un excès de méthanol le mélange contenant de l'eau, obtenu dans l'étape a),
c) on envoie dans une première colonne de distillation le mélange obtenu dans l'étape b),
d) on soutire l'eau du pied de la première colonne de distillation,
e) on évacue le mélange, contenant du méthanol, de l'éther diméthylique et du chlorométhane, apparaissant à la tête de la première colonne de distillation,
f) on envoie à une colonne de distillation extractive le mélange obtenu dans l'étape e),
g) on introduit du méthanol comme agent d'extraction dans la partie supérieure de la colonne de distillation extractive,
h) on soutire du pied de la colonne de distillation extractive un mélange de méthanol et d'éther diméthylique,
i) on évacue le chlorométhane de la tête de la colonne de distillation extractive,
k) on envoie à une seconde colonne de distillation le mélange prélevé dans l'étape h),
l) on soutire le méthanol du bas de la seconde colonne de distillation,
m) on prélève l'éther diméthylique à la tête de la seconde colonne de distillation,
n) on envoie une partie du méthanol obtenu dans l'étape l) aux réactions décrites dans les étapes a) et b), et
o) on envoie la partie restante du méthanol obtenu dans l'étape l) à l'addition de méthanol décrite dans l'étape g), dans la partie supérieure de la colonne de distillation extractive.

2. Procédé selon la revendication 1, caractérisé en ce qu'une partie du courant de produit obtenu dans l'étape m) est envoyée au réactions décrites dans les étapes a) et b).

3. Procédé selon la revendication 1 ou 2, caractérisé en ce que les produits de tête obtenus dans les étapes i) et m) sont condensés avant leur nouvelle utilisation.

4. Procédé selon l'une des revendications 1 à 3, caractérisé en ce que le mélange obtenu dans l'étape e) est condensé et envoyé en phase liquide à la suite du traitement.

5. Procédé selon l'une des revendications 1 à 4, caractérisé en ce que le mélange réactionnel apparaissant à l'état gazeux pendant l'envoi décrit dans l'étape c) est d'abord condensé et ensuite vaporisé de nouveau.

6. Procédé selon l'une des revendications -1 à 5, caractérisé en ce que la distillation extractive ayant lieu après l'étape f) est effectuée sous des pressions comprises entre 1 et 25 bars.

7. Procédé selon l'une des revendications 1 à 6, caractérisé en ce que les colonnes utilisées ont la configuration de colonnes à garnissage ou corps de remplissage.

8. Procédé selon l'une des revendications 1 à 7, caractérisé en ce que, dans l'étape g), le méthanol pour extraction est introduit à une température comprise entre 5 et 50°C dans la colonne de distillation extractive.

9. Procédé selon l'une des revendications 1 à 8, caractérisé en ce que l'on chauffe le mélange décrit dans l'étape k), avant l'introduction dans la seconde colonne de distillation, et en ce que l'on refroidit le méthanol mentionné dans l'étape o), avant l'introduction dans la colonne de distillation extractive.

10. Procédé selon la revendication 9, caractérisé en ce que le chauffage du mélange décrit dans l'étape k) s'effectue dans un échangeur thermique, en ce que le refroidissement du méthanol mentionné dans l'étape o) s'effectue également dans l'échangeur thermique, et en ce que l'on refroidit le méthanol dans un refroidisseur.

11. Procédé selon l'une des revendications 10, caractérisé en ce que la réaction décrite dans les étapes a) et b) est effectuée à l'aide d'un catalyseur.

12. Procédé selon l'une des revendications 1 à 11, caractérisé en ce que les excès de produit de départ mentionnés dans les étapes a) et b) vont jusqu'à 20 % des quantités stoechiométriques requises pour la réaction.

13. Procédé selon l'une des revendications 1 à 11, caractérisé en ce que
1) une partie du mélange obtenu dans l'étape e) est renvoyée à la tête de la première colonne de distillation, et/ou
2) le produit obtenu dans l'étape l) est en partie envoyé à la tête de la colonne de distillation extractive, et/ou
3) une partie du produit obtenu dans l'étape m) est renvoyée à la tête de la seconde colonne de distillation.
